(19) Europäisches Patentamt
European Patent Office
Office européen des brevets

(11) Publication number: **0 323 645 B1**

(12) **EUROPEAN PATENT SPECIFICATION**

(45) Date of publication of patent specification: **31.03.93**

(51) Int. Cl.[5]: **A61K 35/80**, //C12N5/02

(21) Application number: **88121857.2**

(22) Date of filing: **29.12.88**

The file contains technical information submitted after the application was filed and not included in this specification

(54) **Extract of Chlorella and use thereof.**

(30) Priority: **05.01.88 JP 63107/88**

(43) Date of publication of application:
**12.07.89 Bulletin 89/28**

(45) Publication of the grant of the patent:
**31.03.93 Bulletin 93/13**

(84) Designated Contracting States:
**CH DE ES FR GB IT LI NL**

(56) References cited:
**FR-M- 5 576**
**JP-A-62 123 128**

**COMMONWEALTH AGRICULTURAL BUREAU, 1973, no. 74765671; N. GETYA: "Chlorella, a source of biological active substance"**

**CHEMICAL ABSTRACTS, vol. 108, 1988, page 545, abstract no. 73748h, Columbus, Ohio, US**

**CHEMICAL ABSTRACTS, vol. 68, 1968, page 7373, abstract no. 76018m, Columbus, Ohio, US; W. KOWALLIK: "The effect of potassium iodide on light-enhanced endogenous respiration of algae"**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Sawai, Kiichi c/o Sanwa Kagaku Kenkyusho Co.**
**35, Higashi-sotobori-cho Higashi-ku**
**Nagoya Aichi-ken(JP)**
Inventor: **Kurono, Masayasu c/o Sanwa Kagaku Kenkyusho Co.**
**35, Higashi-sotobori-cho Higashi-ku**
**Nagoya Aichi-ken(JP)**
Inventor: **Hara, Kazue c/o Sanwa Kagaku Kenkyusho Co.**
**35, Higashi-sotobori-cho Higashi-ku**
**Nagoya Aichi-ken(JP)**
Inventor: **Jomori, Takahito c/o Sanwa Kagaku Kenkyusho Co.**
**35, Higashi-sotobori-cho Higashi-ku**
**Nagoya Aichi-ken(JP)**

Inventor: **Mitani, Takahiko c/o Sanwa Kagaku Kenkyusho Co.**
**35, Higashi-sotobori-cho Higashi-ku**
**Nagoya Aichi-ken(JP)**
Inventor: **Uchida, Keiichi**
**1-23-13, Shiboku-honcho Miyamae-ku**
**Kawasaki-shi Kanagawa-ken(JP)**
Inventor: **Tanaka, Hiromi**
**1312-43, Shirane-cho Asahi-ku**
**Yokohama-shi Kanagawa-ken(JP)**


(74) Representative: **Wächtershäuser, Günter, Dr.**
**Tal 29**
**W-8000 München 2 (DE)**

# EP 0 323 645 B1

## Description

The present invention relates to a process for obtaining an extract of fresh-water chlorella cultivated in a medium containing an iodide or iodate, and to a pharmaceutical composition comprising such extract.

The chlorella is one of unicellular chlorophycerae, and is excellent as one of sources for human and other animal food, since it shows an excellent utilization efficiency of sun-beam and other light energy, shows a rapid proliferation than other conventional plants, and contains various nutritive materials, such as proteins, lipids, carbohydrates, vitamins or minerals. Further, it has been known that a biologically active substance(s) contained in the chlorella shows an action for accelerating the growth of bacterias, animals and plants. The chlorella has also been expected from its pharmacological activities, namely utilization as an effective ingredient for medicines to cure tumor on digestive organs, hypercholesterinemia or cancers. These biological activities of the chlorella have been studied, in connection with its cellular proliferation factor.

The chlorella is classified into a marine one as natural product and a fresh-water one obtained through a cultivation, but the latter type chlorella occupies an absolute majority in the market, since the former type chlorella has a problem in its productivity.

The cultivation of chlorella in open tanks with natural light in a nutritive layer is described in COMMONWEALTH AGRICULTURAL BUREAU, 1973, No. 74765671. Further, this document discloses that the suspension of chlorella when fed to pigs increased the average daily gain and reduced the feed intake/kg gain.

The inventors have studied from various view points on the marine and fresh-water chlorellas to find that the usually marketed fresh-water chlorella contains substantially no iodine component but the marine cholorella contains a remarkable amount of iodine component which shall be considered as taken from marine-water. Therefore, they have further studied on cultivation of the fresh-water cholorella in a medium added an iodine component to obtain the chlorella containing therein the iodine component and its use for an anti-cancer drug [Jap. Pat. No. 59 - 192083 (A) (Jap. Pat. Appln. No. 65213/1983) and Jap. Pat. No. 62 - 123128 (A) (Jap. Pat. Appln. No. 162144/1986 divided from the Jap. Pat.

It shall be estimated that an extract obtained from the fresh-water chlorella containing therein iodine component may show biological activity and other useful effects different from those of the conventional cellular proliferation factor derived from the fresh-water chlorella containing substantially no iodine component.

A basic object of the present invention lies in obtaining an extract from the fresh-water chlorella containing therein iodine component to elucidate its physico-chemical properties and pharmaceutical activities.

The concrete object of the invention is to provide a process for obtaining the extract.

Another object of the invention is to provide a pharmaceutical composition containing the extract.

According to the invention, a process for obtaining an extract from a fresh-water chlorella is provided, which comprises steps of cultivating the fresh-water chlorella in a medium containing iodine ion of at least 100 ppm under a light-beam, separating the chlorella from the medium, adding water to the separated chlorella to heat for at least several minuted at 100°C, separating insoluble materials to obtain the extract as filtrate, which essentially consists of iodine of at least 100 ppm, saccharides of at least 10 % by weight, proteins of at least 10 % by weight, and nucleic acids of at lease 4.5 mg/ml. The aqueous extract may be freeze-dried for preservation.

As the fresh-water chorella to be employed for the invention, chlorella pyrenoidosa. chlorella burgaris, chlorella eribrides, chorella cenedesumus, chlorela cramidomonas, chorella cerenastorum and the like may be listed, but the chorella pyrenoidosa, chorella burgaris and chorella eribrides are more preferable. The chorella may be subjected to 2 stage cultivation system consisting of a first or pre-cultivation and a second or main cultivation. The pre-cultivation can be carried out by planting the chlorella in a medium, while stirring the medium by introducing air containing 2% $CO_2$, under radiation of white light-beam (several thousands lux, for instance 5000 lux) for several days (for instance, 5 days) at a temperature of about 25°C. The main cultivation can be carried out with use of another medium containing an acetate as main nutritive source and iodine ion of at least 100ppm, for instance 300ppm. The pre-cultivated chlorella is planted in the medium, so that its packed cell volume becomes 2 to 4 $\mu$l/ml. The cultivation requires about 4 to 7 days at a temperature ranging from 25 to 30°C, under radiation of white light beam (several thousands lux, for instace 3500 lux) with stirring, while introducing air of 3 litres/min. The artificial light radiation may be changed to natural light of sun-beam. The medium is to be controlled in its pH in a range of 6.0 to 8.0, by adding an acetate supply medium.

3

The chlorella extract according to the invention can be employed as an effective ingredient for medicines or as a food additive. In case of preparing the medicines, there is no limitation in its medicine form and thus it may be made into one for oral or non-oral route dosing, with use of a conventional pharmaceutical carrier or excipient, namely powder, granules, tablets, sugar-coated tablets, capsules and the like solid form, or solution, suspension, emulsion or the like liquid form. If necessary, conventional additives such as auxiliary, stabilizer, wetting agent, emulsifier, buffer and the like may be composed.

The invention will now be further explained with reference to an Example for obtaining an extract according to the invention and pharmacological Test Examples, which will refer to drawings wherein

Fig. 1 shows an elusion profile of an extract according to the invention, when the extract was subjected to a gel-filtration with use of Sephadex G-25 column;

Fig. 2 is a graph showing results of molecular weight determination of proteins in Fraction A shown in Fig. 1, according to SDS polyacryloamide-gel electrophoresis method, together with various molecular weight markers;

Fig. 3 is a graph showing concentration of $T_4$ in serum on various test rat groups, when 3 weeks were lapsed from beginning of the test;

Fig. 4 is a graph similar to that in Fig. 3, excepting that 6 weeks were lapsed from beginning of the test;

Fig. 5 is a graph showing results of size determination of a thyroid gland on various test rat groups, when 6 weeks were lapsed from beginning of the test; and

Fig. 6 is a graph showing results of wet weight determination of a thyroid gland on various test rat groups, when 6 weeks were lapsed from beginning of the test.

Example

(1) Cultivation of fresh-water chlorella

(a) Aseptic cultivation

A sample of a fresh-water chlorella (Chlorella pyrenoidosa W) was plated in a medium having a composition as shown in following Table 1 and pre-sterilized through a thermal treatment at 120 °C for 15 minutes, prior to a pre-cultivation. The pre-cultivation was carried out at 25°C for 5 days, while stirring the medium with 2% $CO_2$ containing air and under radiation of white light-beam (5000 lux).

Table 1

| (Medium for pre-cultivation) | |
|---|---|
| Components | Amounts |
| $KNO_3$ | 5.0g |
| $MgSO_4 \cdot 7H_2O$ | 2.5g |
| $KH_2PO_4$ | 1.25g |
| $FeSO_4 \cdot 7H_2O$ | 5.0gmg |
| EDTA | 7.0mg |
| ARNON $A_5$ (*) | 1.0ml |
| Distilled water | 1000ml |
| pH | 6.5 |

In the Table,
(*) : Prepared by adding and mixing 2.86g of $H_3BO_3$, 1.81g of $MnCl_2 \cdot 4H_2O$, 0.22g of $ZnSO_4 \cdot 7H_2O$, 0.08g of $CuSO_4 \cdot 5H_2O$, 0.021g of $Na_2MoO_4$, 1000ml of pure water, and 1 drop of conc-$H_2SO_4$.

After completion of the pre-cultivation, 4 litres of a basic medium having a composition as given in following Table 2 were charged in a jar fermenter (volume of 7 litres) and potassium iodate ($KIO_3$) was charged therein, so that iodine ion in the basic medium becomes 300rpm. In the fermenter, the pre-cultivated chlorella was charged, so that its packed cell volume becomes 2 to 4 ($\mu$l/ml). This main cultivation was carried automatically adding an acetate supply medium having composition as given in following Table 3, to maintain pH in a range of 6.2 to 6.5.

4

EP 0 323 645 B1

| Intensity of illumination | 3500 lux, |
|---|---|
| Air feeding ratio | 3 litres/minute, and |
| Velocity of rotation | 300rpm. |

Table 2

| (Basic medium for main cultivation) | |
|---|---|
| Components | Amounts |
| Urea | 300mg |
| $KH_2PO_4$ | 150mg |
| $MgSO_4 \cdot 7H_2O$ | 150mg |
| $FeSO_4 \cdot 7H_2O$ | 10mg |
| ARNON $A_5$ (*) | 1ml |
| Distilled water | 1000ml |
| pH | 6.7 |

* : See Table 1

Table 3

| (Acetate supply medium) | |
|---|---|
| Components | Amounts |
| Acetic acid | 400g |
| EDTA-2Na | 1.4g |
| $FeSO_4 \cdot 7H_2O$ | 1.0g |
| $MgSO_4 \cdot 7H_2O$ | 12g |
| Urea | 50g |
| $KH_2PO_4$ | 24g |
| ARNON $A_5$ (*) | 2ml |
| Distilled water | 1000ml |

* : See Table 1

The cultivated chlorella was subjected to centrifugal treatment and then the resulting wet cake was freeze dried. The resulting powdered chlorella was wet-decomposed with use of a mixed solution of chromic acid and sulfuric acid and the resulting formed iodine was reacted with phosphorous acid to make it into iodic acid which was absorbed into sodium hydroxide solution through distillation. A contacting reaction of thiocyanic acid (III) with iodine was conducted with use of the iodic acid absorbed solution to find that the fresh-water chlorella intook iodine of 32ppm as an amount of iodine ion, through the cultivation period of time of 7 days.

(b) Outdoor cultivation

In a circular cultivation pool with a stirrer and having a diameter of 19 meters, the basic medium as shown in Table 2 of said Item (a) was charged in an amount of that the depth of the medium becomes 15cm. After planted in the medium the fresh-water chlorella of the kind same with that in Item (a) to make its packed cell volume into $3\mu l/ml$, potassium iodate was added in the pool, so that the concentration thereof becomes 100ppm as that of iodine ion, and then the acetate supply medium as shown in Table 3 of said Item (a) was added continuously to maintain pH of the medium throughout in the pool in a range of 6.5 to 8.5. The depth of the medium in the pool was increased by 5cm in every other day and it finally made into 30cm for cultivation period of time of 7 days. When the depth was increased, potassium iodate was added to always keep the concentration of iodine ion at 100ppm. A packed cell volume of the chlorella at

5

the last day of cultivation period of time was 12μl/ml and a concentration of iodine intaken by the chlorella was 57.7ppm as an amount of iodine ion.

(2) Extraction operation

The mixture of medium and cultivated chlorella, completed the 7 day main cultivation period of time in Item 1-a was subjected to centrifugal treatment to obtain a wet-cake of the chlorella. 640ml of distilled water were added to 320g of the wet-cake and heated for 10 minutes at 100°C. After cooled to the room temperature, the mixture was subjected to centrifugal treatment (5000rpm, 10 minutes) to remove chlorella residue and to obtain an aqueous extract. The aqueous extract was freeze-dried to obtain the extract (this may be referred to hereinafter as --A-CGF--) in the form of powder (Yield : 24g).

11g of the powdered A-CGF were dissolved in water and the resulting solution was subjected to a gel-filtration with use of Sephadex G-25 column (4 x 100cm) and elute was collected in each fraction by 70ml.

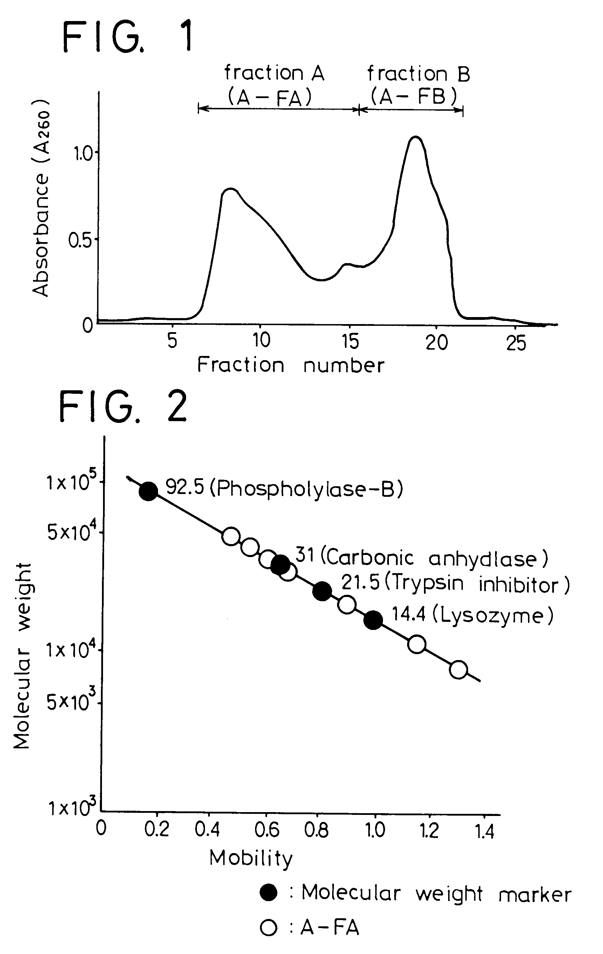

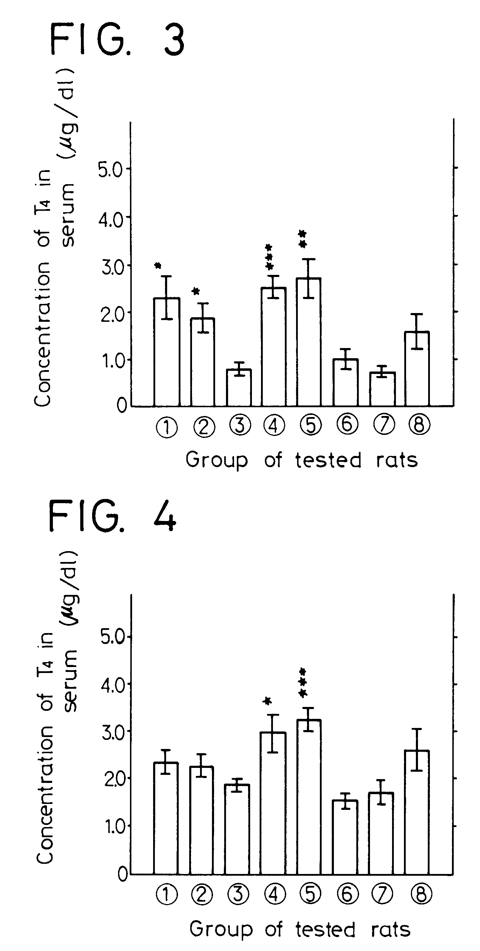

Results are shown in Fig. 1. As apparently seen from the Fugure, fractions are classified into 2 groups, namely Fraction Group A (hereinafter referred to --A-FA--) of higher molecular weight fractions and Fraction Group B (hereinafter referred to --A-FB--) of lower molecular weight fractions. On the A-FA, SDS polyacryloamide electrophoresis analysis showed that it contains 7 kind proteins (MW : 46100, 40000, 34000, 30000, 18000, 10500 and 7000), in which main proteins are those having molecular weight of 34000, 30000, 18000 and 7000 (see Fig. 2). While, on the A-FB, Lowry method showed as positive, but according to SDS polyacryloamide electrophoresis analysis, it contains no protein having molecular weight higher than 7000.

Further, contents of iodine (according to the method as described in Item 1-a), saccharides (according to phenol-sulfuric acid method), proteins (according to Lowry method) and nucleic acids (according to absorbance measurement of 0.01% solution, at $A_{260}$) in the A-CGF, A-FA and A-FB as well as controls of powdered extract obtained from the fresh-water chlorella similar to that to A-CGF but containiong no iodine (this control extract is hereinafter referred to as --C-CGF--), a higher molecular weight fraction group similar to the A-FA but in the C-CGF (this fraction group is referred to as --C-FA--) and a lower molecular weight fraction group similar to A-FB but in the C-CGF (this fraction group is referred to as --C-FB--) were determined. Results are shown in following Table 4.

Table 4

|  | Iodine (ppm) | Saccharaides (wt.%) | Proteins (wt.%) | Nucleic acids (mg sample/ml) |
|---|---|---|---|---|
| A-CGF | 297 | 23.4 | 33.6 | 7.5 |
| A-FA | 19 | 27.4 | 30.5 | 4.9 |
| A-FB | 729 | 18.6 | 12.8 | 9.0 |
| C-CGF | 0 | 22.0 | 44.6 | 4.0 |
| C-FA | 0 | 21.6 | 46.2 | 2.6 |
| C-FB | 0 | 16.2 | 17.9 | 6.6 |

Pharmacological Test Example 1

(Anti-tumor activity)

Each of the A-CGF and C-CGF was dissolved in distilled water and thermally treated (100°C, 5 minutes) for sterilization. The resulting solution was injected intraperitoneally to ddy mice (male, age of 5 weeks) over 5 days in dosing amount of 10, 100 and 1000mg/kg/day. On the day next to the final dosing day, $1 \times 10^4$ or $1 \times 10^5$ cells of Sarcoma 180 as an ascites cancer cell were transplanted into an abdominal canal of each mouse and life or death of the animals were observed over 40 days from the transplantation. Results are shown in following Table 5. From the Table, the A-CGF shows a noticiable anti-tumor action on dose of 10mg/kg, in case of $1 \times 10^4$ cancer cell transplantation and doses of 10mg/kg and 100mg/kg, in case of $1 \times 10^5$ cancer cell transplantation, although it somewhat weak, but there is found no anti-tumor action on the C-CGF.

6

## Table 5

| Test group | dose (mg/kg) | Number of transplanted cancer cells | | | | | |
|---|---|---|---|---|---|---|---|
| | | $1 \times 10^4$ | | | $1 \times 10^5$ | | |
| | | Days of life prolongation (mean±S.E.) | ratio of life prolongation (%) | living mouse after 40 days (head) | Days of life prolongation (mean±S.E.) | ratio of life prolongation (%) | living mouse after 40 days (head) |
| Control | Physical Saline | 23.1±0.63 | 100 | 0/13 | 21.9±0.65 | 100 | 0/13 |
| C-CGF (Ltr no.1) | 10 | 24.7±0.88 | 107 | 0/10 | 23.0±0.84 | 105 | 0/10 |
| | 100 | 23.3±0.73 | 101 | 0/10 | 23.5±0.72 | 107 | 0/10 |
| | 1000 | 21.7±0.58 | 94 | 0/10 | 21.6±0.96 | 99 | 0/10 |
| C-CGF (Lot no.4) | 10 | 25.2±1.06 | 109 | 0/10 | 21.9±0.74 | 100 | 0/10 |
| | 100 | 23.5±1.22 | 102 | .0/10 | 25.0±1.52 | 114 | 0/10 |
| | 1000 | 22.4±1.09 | 97 | 0/10 | 20.5±0.78 | 94 | 0/10 |
| A-CGF | 10 | 26.1±1.34 | 113 | 0/10 | 25.4±1.44 | 116 | 0/10 |
| | 100 | 24.4±1.26 | 106 | 0/10 | 24.3±0.98 | 111 | 0/10 |
| | 1000 | 23.9±1.46 | 104 | 0/10 | 20.3±0.65 | 93 | 0/10 |

EP 0 323 645 B1

Pharmacological Test Example 2

(Action for preventing hyperchoresterolemia due to functional reduction of thyroid gland)

An action of the A-CGF to hypercholesterolemia due to hypthyreosis to be caused by intaking of propylthiouracil (referred to --PTU--) was compared with that of the C-CGF.

SD rats (male, age of 7 weeks) were classified into following groups, to give following food.

① Normal food,
② Low iodine food,
③ Low iodine food + PTU (62.5μg)
④ Low iodine food + PTU (62.5μg) + A-CGF (12μg)
⑤ Low iodine food + PTU (62.5μg) + A-CGF (60μg)
⑥ Low iodine food + PTU (62.5μg) + C-CGF (12μg)
⑦ Low iodine food + PTU (62.5μg) + C-CGF (60μg)
⑧ Low iodine food + PTU (62.5μg) + $KIO_3$ (60μg)

Note : The bracketed numeral means a dose/rat/day.

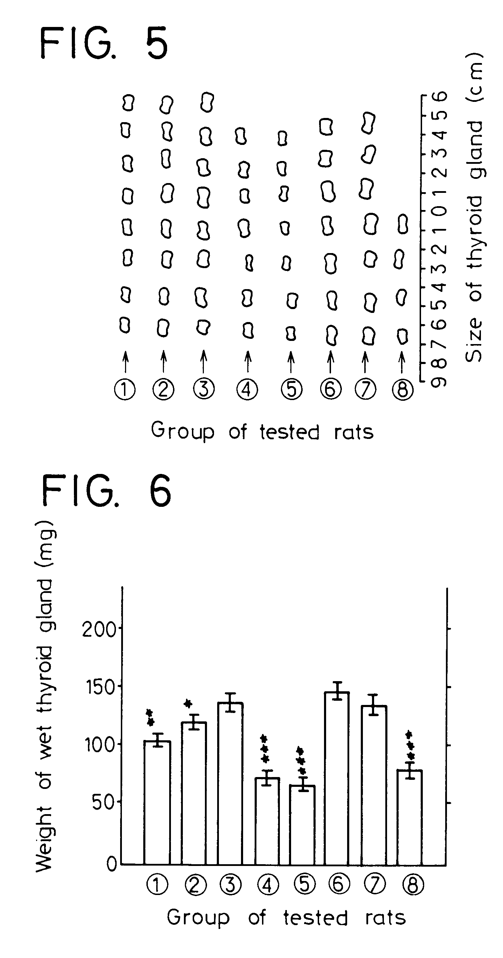

The animals in each group were breeded over 6 weeks. By the way of breeding period of time, $T_4$ concentration in blood at 3rd and 6th week, as well as size and wet weight of thyroid gland at 6th week were determined to obtain results as shown in Figs. 3 to 6, respectively.

In the Figures, marks of *, ** and *** show a fact that there is noticeable difference, based on the control of third group (③) under following ratio of risk.

| * | $P < 0.05$ |
|---|---|
| ** | $P < 0.01$ |
| *** | $P < 0.001$ |

As seen from the Figures, the A-CGF shows a noticiable prevention of the hypercholesterolemia, but the C-CGF does not show such prevention.

## Claims

1. A process for obtaining an extract from a fresh-water chlorella, which comprises steps of cultivating the fresh-water chlorella in a medium containing iodine ion of at least 100 ppm under a light-beam, separating the chlorella from the medium, adding water to the separated chlorella to heat for at least several minutes at 100°C, separating insoluble materials to obtain the extract as filtrate, which essentially consists of iodine of at least 100 ppm, saccharides of at least 10 % by weight, proteins of at least 10 % by weight, and nucleic acids of at least 4.5 mg/ml.

2. A process as claimed in Claim 1, further comprising a step for freeze drying to make the aqueous extract into a powdered extract.

3. A pharmaceutical composition for use in human or veterinary medicine, which comprises an effective amount of the extract of Claim 1 in association with a pharmaceutical carrier or excipient.

## Patentansprüche

1. Ein verfahren zum Herstellen eines Extraktes aus einer Frischwasser-Chlorella, umfassend die Stufen des Kultivierens der Frischwasser-Chlorella in einem Medium, das mindestens 100 ppm Jodionen enthält, unter Belichtung, Abtrennen der Chlorella aus dem Medium, Hinzugeben von Wasser zu der abgetrennten Chlorella, um sie mindestens einige Minuten lang auf 100°C zu erhitzen, Abtrennen unlöslicher Materialien, um den Extrakt als Filtrat zu erhalten, das im wesentlichen aus mindestens 100 ppm Jod, mindestens 10 Gew.-% Sacchariden, mindestens 10 Gew.-% Proteinen und mindestens 4,5 mg/ml Nukleinsäuren besteht.

2. Ein Verfahren nach Anspruch 1, weiter umfassend eine Stufe des Gefriertrocknens, um aus dem wässerigen Extrakt einen pulverförmigen Extrakt zu machen.

3. Pharmazeutische Zubereitung zum Gebrauch in der Human- oder Veterinär-Medizin, die eine wirksame Menge des Extraktes nach Anspruch 1 zusammen mit einem pharmazeutischen Träger oder Binder umfaßt.

**Revendications**

1. Procédé d'obtention d'un extrait de chlorelle d'eau douce, qui comprend les étapes de :
   - culture de la chlorelle d'eau douce dans un milieu contenant l'ion iode à raison d'au moins 100 ppm, sous un faisceau lumineux ;
   - séparation de la chlorelle du milieu ;
   - addition d'eau à la chlorelle séparée, pour chauffer pendant au moins plusieurs minutes à 100°C ;
   - séparation des matières insolubles pour obtenir l'extrait en tant que filtrat, lequel consiste essentiellement en iode à raison d'au moins 100 ppm d'iode, saccharides à raison d'au moins 10% en poids, protéines à raison d'au moins 10% en poids, et acides nucléiques à raison d'au moins 4,5 mg/ml.

2. Procédé selon la revendication 1, qui comprend en outre une étape de lyophilisation pour amener l'extrait aqueux sous la forme d'un extrait pulvérulent.

3. Composition pharmaceutique pour utilisation en médecine humaine ou vétérinaire, qui comprend une quantité efficace de l'extrait tel que défini à la revendication 1, en association avec un support ou excipient pharmaceutique.

# FIG. 1

# FIG. 2

# FIG. 3

# FIG. 4

# FIG. 5

Size of thyroid gland (cm)

Group of tested rats

# FIG. 6

Weight of wet thyroid gland (mg)

Group of tested rats